# EUROPEAN PATENT SPECIFICATION

(11) **EP 1 912 687 B1**
(45) Date of publication and mention of the grant of the patent: **17.03.2010**
(21) Application number: 05802493.6
(22) Date of filing: 10.11.2005
(51) Int. Cl.: A61M 1/10, A61L 27/06

(54) **Blood pump, in particular pneumatic heart assist device**
Blutpumpe, insbesondere pneumatische Vorrichtung zur kardialen Unterstützung
Pompe sanguine, en particulier dispositif d'assistance cardiaque pneumatique

(30) Priority: 11.11.2004 PL 37114704
(43) Date of publication of application: 23.04.2008
(73) Proprietor: Fundacja Rozwoju Kardiochirurgii, 41-800 Zabrze (PL); Instytut Metalurgii i Inzynierii Materialowej PAN, 30-059 Krakow (PL); Joanneum Research Forschungsgesellschaft mbH, 8010 Graz (AT)
(72) Inventor: EBNER, Reinhold, A-8700 Leoben (AT); KUSTOSZ, Roman, PL-41-800 Zabrze (PL); LACKNER, Jürgen, M., A-8793 Gai (AT); MAJOR, Boguslaw, PL-30-837 Krakow (PL); RELIGA, Zbigniew, PL-00-391 Warszawa (PL); STOLARZEWICZ, Bogdan, PL-40-057 Katowice (PL); WALDHAUSER, Wolfgang, A-8700 Leoben (AT); WIERZCHON, Tadeusz, PL-02-642 Warszawa (PL)
(74) Representative: Malcherek, Piotr
(86) International application number: PCT/PL2005/000073
(87) International publication number: WO 2006/052153

(56) References cited:
- EP-A- 1 087 034
- FR-A- 2 784 585

## Description

The present invention relates to a blood pump, in particular pneumatic heart assist device, used as a partially or completely implantable device for treatment of human heart diseases.

Recently heart assist devices or blood pumps have been more and more employed in treatment of human heart diseases, in particular to assist the blood flow or to produce it. They are used as extracorporeal or implantable devices. In most cases such pumps or devices are employed to help the inefficient heart working functions for a limited period of time, lasting from a few days to several weeks.

There are known extracorporeal pneumatic heart assist devices incorporated into human blood-vascular system. Inside the body of the device there is an elastic membrane which divides the body into two chambers. The pneumatic chamber of the device is connected with a pneumatic generator. Cyclic pneumatic pressure causes cyclic membrane movements. As a result blood which flows through the blood chamber of the device is stimulated to flow. The blood flow is also controlled by heart valves which are located inside the inlet and outlet ports of the device. Known extracorporeal heart devices, for example the Polish POLVAD, have all or nearly all inside elements, including the membrane and leaflets of the valves made of plastic, in particular of polyurethane. The advantage of using polyurethane is that it is not expensive and is biocompatible with human blood and tissue. Additionally polyurethane is an elastic material, and elements of the device made of polyurethane work efficiently. A disadvantage of using polyurethane is limited fatigue strength of elements made of polyurethane. Such elements, being in persistent contact with blood, execute cyclic movements which causes cyclic mechanical stress. After some period of time this could lead to damages of such elements. Improper working of the device is very dangerous for the patients and can lead to obstruction in blood flow or even fatal damage of the device. This can have fatal consequences for the patient. Apart from this long-term working of elements made of polyurethane causes microcracks on the outside surfaces of such elements, which is called fatigue degradation of the polyurethane chain. Local adhesion of blood platelets as well as calcium depositions could take place during blood contact with such microcracks. As a result such elements can not be used in long-term working devices, in particular in permanent implantable devices.

There are also known partially or completely implantable devices which are very comfortable for the patient since the patient can live normally with such devices. Such devices have their working elements made of titanium. Titanium is known to be a biocompatible and strongly biodegradation resistant material. The disadvantage of using titanium is the fact that it is expensive and non-elastic. If an element, for example a heart valve, is made of titanium, due to the rigidity of the titanium the blood flow is quite different from natural. This can lead to negative local blood flow irregularity, for example cavitations, which can be very dangerous for the patient. The document FR 2 784 585 A discloses a prior art pump.

The aim of the invention is to construct a blood pump which could provide a long-term working facility, which would not be very expensive in manufacturing and which would provide blood flow approximate to blood flow in a natural heart.

According to the invention the selected surfaces of the elastic elements of the pump made of plastic, in particular of polyurethane, are covered with at least two-layer coating with thickness between 5 nm and 500 nm, wherein the base buffer layer of the coating is made of nanocrystalline titanium or titanium based materials, which is covered with at least one layer made of titanium nitride and/or titanium carbonitride, each of them having nanocrystalline structure.

Surprisingly, the elastic elements of the pump covered with such coating have satisfying fatigue strength, while their elastic properties have generally not changed. As an effect, the new elastic elements of the blood pump work efficiently without any damage for a long period of time. The cost of manufacturing such new pump elastic elements is only sightly higher than the cost of manufacturing known pumps, and as a result of the longer working time such heart pumps can be successfully partially or completely implantable into the human body.

In a preferred embodiment all surfaces of the elastic elements of the pump which are dedicated to be in contact with human tissue or blood are covered with at least two-layer coating with thickness between 5 nm and 500 nm, wherein the base buffer layer of the coating is a layer made of nanocrystalline titanium or titanium based materials, which is covered with at least one layer made of titanium nitride and/or titanium carbonitride, each of them having nanocrystalline structure.

In the most preferred embodiment all surfaces of the elastic elements of the pump are covered with at least two-layer coating with thickness between 5 nm and 500 nm, wherein the base buffer layer of the coating is a layer made of nanocrystalline titanium or titanium based materials, which is covered with at least one layer made of titanium nitride and/or titanium carbonitride, each of them having nanocrystalline structure.

In order to increase the mechanical durability of the elastic elements of the pump as well as to increase the biocompatibility of the elastic elements with human blood and/or tissue, the base buffer layer of the coating made of nanocrystalline titanium or titanium based materials is covered with at least one layer made of titanium nitride and one layer made of titanium carbonitride, each of them having nanocrystalline structure.

It is also preferred when the base buffer layer of the coating made of nanocrystalline titanium or titanium based materials is covered alternately with a number of layers made of titanium nitride and a number of layers made of titanium carbonitride.

The base buffer layer of the coating made of nanocrystalline titanium or titanium based materials constitutes between 1 and 50 % of the thickness of the whole coating. Such construction secures in the best possible manner the mechanical stresses distribution in the elastic elements of the pump.

It is also preferred when the thickness of the whole coating is between 20 nm and 100 nm, most preferred 50 nm.

The invention has been described in the following embodiments with reference to the drawing, where fig. 1 is a schematic side view of a segment of the surface of elastic working element of pneumatic heart assist device in the first embodiment, fig. 2 - schematic side view of alike segment in second embodiment and fig. 3 - schematic side view of alike segment in third embodiment.

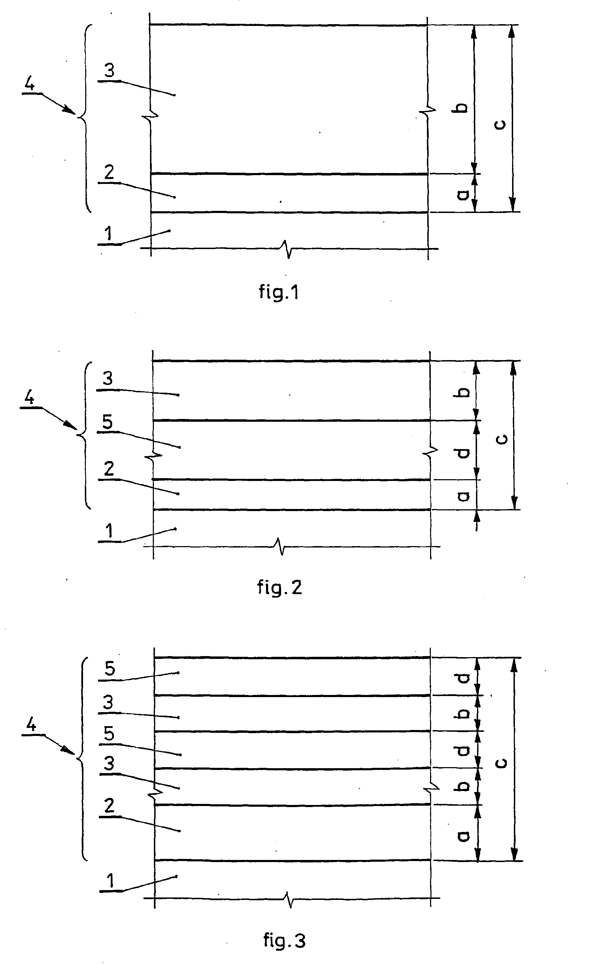

In the first embodiment ( fig. 1 ) elastic elements of pneumatic heart assist device, in particular the membrane and leaflets of heart valves are made of polyurethane 1. The whole outside surface of such elements is covered by laser ablation with a layer made of titanium 2, with thickness a of 20 nm. Layer 2 constitutes a, so called, buffer layer. Titanium layer 2 is covered, also by laser ablation, with a layer made of titanium nitride ( Ti-N ) 3, with thickness b of 80 nm. The thickness c of the whole coating 4 which covers the polyurethane elements 1 is 100 nm.

In the second embodiment ( fig. 2 ) the whole outside surface of the polyurethane elements 1 is covered, by laser ablation, with a layer made of titanium 2, with thickness a of 40 nm. Layer 2 constitutes a, so called, buffer layer. Titanium layer 2 is covered, also by laser ablation, with a layer made of titanium carbonitride ( Ti-C-N ) 5, with thickness d of 80 nm. Additionally the layer made of titanium carbonitride 5 is covered, also by laser ablation, with a layer made of titanium nitride 3 with thickness b of 80 nm. The thickness c of the whole coating 4 which covers the polyurethane elements 1 is 200 nm.

In the third embodiment ( fig. 3 ) the whole outside surface of the polyurethane elements 1 is covered, by laser ablation, with a layer made of titanium 2, with thickness a of 30 nm. Layer 2 constitutes a, so called, buffer layer. Titanium layer 2 is covered, also by laser ablation, alternately with a number of layers made of titanium nitride 3 with thickness b of 20 nm, titanium carbonitride 5 with thickness d of 20 nm, titanium nitride 3 with thickness b of 20 nm and titanium carbonitride 5 with thickness d of 20 nm. The thickness c of the whole coating 4 which covers the polyurethane elements 1 is 110 nm. All layers 2, 3 and 5 have a nanocrystalline structure.

## Claims

1. Blood pump, in particular pneumatic heart assist device, having main body and elastic working elements, such as a membrane and heart valves, wherein selected elements are made of plastic, in particular of polyurethane, **characterised by** the fact that the selected surfaces of the elastic elements ( 1 ) of the pump are covered with at least two-layer coating ( 4 ) with thickness ( c ) between 5 nm and 500 nm, wherein the base buffer layer of the coating is made of nanocrystalline titanium or titanium based materials ( 2 ), which is covered with at least one layer made of titanium nitride ( 3 ) and/or titanium carbonitride ( 5 ), each of them having nanocrystalline structure.

2. Blood pump according to claim 1, **characterised by** the fact that all surfaces of the elastic elements ( 1 ) of the pump which are dedicated to be in contact with human tissue or blood are covered with at least two-layer coating ( 4 ) with thickness ( c ) between 5 nm and 500 nm, wherein the base buffer layer of the coating is made of nanocrystalline titanium or titanium based materials ( 2 ), which is covered with at least one layer made of titanium nitride ( 3 ) and/or titanium carbonitride ( 5 ), each of them having nanocrystalline structure.

3. Blood pump according to claim 2, **characterised by** the fact that all surfaces of the elastic elements ( 1 ) of the pump are covered with at least two-layer coating ( 4 ) with thickness ( c ) between 5 nm and 500 nm, wherein the base buffer layer of the coating is made of nanocrystalline titanium or titanium based materials ( 2 ), which is covered with at least one layer made of titanium nitride ( 3 ) and/or titanium carbonitride ( 5 ), each of them having nanocrystalline structure.

4. Blood pump according to claim 1 or 2 or 3, **characterised by** the fact that the base buffer layer of the coating made of nanocrystalline titanium or titanium based materials ( 2 ) is covered with at least one layer made of titanium nitride ( 3 ) and one layer made of titanium carbonitride ( 5 ), each of them having nanocrystalline structure.

5. Blood pump according to claim 4, **characterised by** the fact that the base buffer layer of the coating made of nanocrystalline titanium or titanium based materials ( 2 ) is covered alternately with a number of layers made of titanium nitride ( 3 ) and a number of layers made of titanium carbonitride ( 5 ).

6. Blood pump according to claim 1 or 2 or 3, **characterised by** the fact that the base buffer layer of the coating made of nanocrystalline titanium or titanium based materials ( 2 ) constitutes between 1 and 50 % of the thickness ( c ) of the whole coating ( 4 ).

7. Blood pump according to claim 1 or 2 or 3, **characterised by** the fact that the thickness ( c ) of the whole coating ( 4 ) is between 20 nm and 100 nm, most preferred 50 nm.

## Patentansprüche

1. Blutpumpe, insbesondere die pneumatische Herzhilfspwnpe, mit einem Gehäuse und flexiblen Betriebselementen, wie Membran und Herzklappen, wobei die ausgewählten Pumpenelemente aus Kunststoffen, insbesondere aus Polyurethanen, gefertigt sind, **gekennzeichnet dadurch, dass** die ausgewählten Oberflächen der flexiblen Pumpenelemente /1/ mit mindestens zweischichtigem Außenüberzug /4/ mit der Dicke /c/ von 5 nm bis 500 nm beschichtet sind, wobei die Puffergrundschicht stellt eine aus dem nanokristalischen Titanium bzw. aus auf Titanium /2/ basierten Verbindungen ausgeführte Schicht dar, die mit mindestens einer Schicht Titaniumnitrid /3/ und/oder Titaniumcarbonitrid /5/ überzogen ist, wobei jede dieser Schichten die nanokristalische Struktur aufweiset.

2. Blutpumpe nach Anspruch 1 **gekennzeichnet dadurch, dass** die sämtlichen Oberflächen der flexiblen Pumpenelementelemente /1/, die mit Menschengeweben bzw. mit Blut in Kontakt treten, einen mindestens zweischichtigen Außenüberzug /4/ mit der Dicke /c/ von 5 nm bis 500 nm haben, wobei die Pufferschicht stellt eine aus dem nanokristalischen Titanium bzw. aus auf Titanium /2/ basierten Verbindungen ausgeführte dar, die mit mindestens einer Schicht Titaniumnitrid /3/ und/oder Titaniumcarbonitrid /5/ überzogen ist, wobei jede dieser Schichten die nanokristalische Struktur aufweist.

3. Blutpumpe nach Anspruch 2 **gekennzeichnet dadurch, dass** die sämtlichen Oberflächen der flexiblen Pumpenelementelemente /1/ einen mindestens zweischichtigen Außenüberzug /4/ mit der Dicke /c/ von 5 nm bis 500 nm haben, wobei die Puffergrundschicht stellt eine aus dem nanokristalischen Titanium bzw. aus auf Titanium /2/ basierten Verbindungen ausgeführte Schicht dar, die mit mindestens einer Schicht Titaniumnitrid /3/ und/oder Titaniumcarbonitrid /5/ überzogen ist, wobei jede dieser Schichten die nanokristalische Struktur aufweist.

4. Blutpumpe nach Anspruch 1 oder 2 der 3 **gekennzeichnet dadurch, dass** die aus nanokristalischen Titanium bzw. aus auf Titanium /2/ basierten Verbindungen ausgeführte Pufferschicht mit mindestens einer Schicht aus Titaniumnitrid /3/ sowie mit mindestens einer Schicht aus Titaniumcarbonitrid /5/ überzogen ist, wobei jede dieser Schichten die nanokristalische Struktur ausweist.

5. Blutpumpe nach Anspruch 4 **gekennzeichnet dadurch, dass** die aus nanokristalischen Titanium bzw. aus auf Titanium /2/ basierten Verbindungen ausgeführte Pufferschicht wechselweise mit einigen Schichten aus Titaniumnitrid /3/ und mit einigen Schichten aus Titaniumcarbonitrid beschichtet ist.

6. Blutpumpe nach Anspruch 1 der 2 oder 3 **gekennzeichnet dadurch, dass** die aus nanokristalischen Titanium bzw. aus auf Titanium /2/ basierten Verbindungen ausgeführte Pufferschicht 1 bis 50% der gesamten Dicke /c/ des ganzen Außenüberzugs /4/ aufweist.

7. Blutpumpe nach Anspruch 1 oder 2 der 3 **gekennzeichnet dadurch, dass** die Dicke /c/ des Außenüberzugs /4/ zwischen 20 mm bis 100 nm, am günstigsten 50 nm, beträgt.

## Revendications

1. Pompe sanguine, en particulier dispositif d'assistance cardiaque pneumatique, équipée d'un corps et d'éléments actifs flexibles tels qu'une membrane et des valves cardiaques, dont certains éléments sont faits en matières plastiques, notamment en polyuréthane, **caractérisée en ce que** certaines surfaces des éléments flexibles ( 1 ) de la pompe sont couvertes d'un revêtement extérieur ( 4 ) au minimum à double couche, d'une épaisseur ( c ) de 5 nm à 500 nm, la couche tampon de base étant constituée d'une couche en titane nanocristallin ou de composés à base de titane ( 2 ), celle-ci étant couverte au minimum d'une couche de nitrure de titane ( 3 ) et/ou d'une couche de carbonitrure de titane ( 5 ), chacune de ces couches ayant une structure nanocristalline.

2. Pompe sanguine, suivant la revendication 1, **caractérisée en ce que** tontes les surfaces des éléments flexibles ( 1 ) de celle-ci pouvant être en contact avec un tissu humain ou avec le sang, sont couvertes d'un revêtement extérieur ( 4 ) au minimum à double couche, d'une épaisseur ( c ) de 5 nm à 500 nm, la couche tampon étant constituée d'une couche en titane nanocristallin ou de composés à base de titane ( 2 ), celle-ci étant couverte au minimum d'une couche de nitrure de titane ( 3 ) et/ou d'une couche de carbonitrure de titane ( 5 ), chacune de ces couches ayant une structure nanocristalline.

3. Pompe sanguine, suivant la revendication 2, **caractérisée en ce que** toutes les surfaces des éléments flexibles ( 1 ) de celle-ci sont couvertes d'un revêtement extérieur ( 4 ) au minimum à double couche, d'une épaisseur ( c ) de 5 nm à 500 nm, la couche tampon étant constituée d'une couche en titane nanocristallin ou de composés à base de titane ( 2 ), celle-ci étant couverte au minimum d'une couche de nitrure de titane ( 3 ) et/ou d'une couche de carbonitrure de titane ( 5 ), chacune de ces couches ayant une structure nanocristalline.

4. Pompe sanguine, suivant la revendication 1 ou 2 ou 3, **caractérisée en ce que** la couche tampon faite en titane nanocristallin ou de composés à base de titane ( 2 ), est couverte au minimum d'une couche de nitrure de titane ( 3 ) et aussi au minimum d'une couche de carbonitrure de titane ( 5 ), chacune de ces couches ayant une structure nanocristalline.

5. Pompe sanguine, suivant la revendication 4, **caractérsée en ce que** que la couche tampon faite en titane nanocristallin ou de composés à base de titane ( 2 ), est couverte en alternance de plusieurs couches de nitrure de titane ( 3 ) et de plusieurs couches de carbonitrure de titane ( 5 ).

6. Pompe sanguine, suivant la revendication 1 ou 2 ou 3, **caractérisée en ce que** la couche tampon faite en titane nanocristallin ou de composés à base de titane ( 2 ) représente 1 à 50 % de l'épaisseur ( c ) de tout le revêtement extérieur ( 4 ).

7. Pompe sanguine, suivant la revendication 1 ou 2 ou 3, **caractérisée en ce** l'épaisseur ( c ) du revêtement extérieur ( 4 ) est comprise dans l'intervalle entre 20 nm et 100 nm, la valeur la plus avantageuse étant 50 nm.
